# EUROPEAN PATENT SPECIFICATION

(11) **EP 3 636 252 B1**
(45) Date of publication and mention of the grant of the patent: **03.11.2021**
(21) Application number: 18798346.5
(22) Date of filing: 10.05.2018
(51) Int. Cl.: A61K 9/08, A61K 47/02, A61K 47/18, A61K 47/10, A61K 47/36, A61K 47/26, A61K 47/04, A61K 31/7056, A61P 27/02

(54) **EYE DROP FOR TREATING EYE DRYNESS**
AUGENTROPFEN ZUR BEHANDLUNG VON TROCKENEN AUGEN
GOUTTE OPHTALMIQUE POUR LE TRAITEMENT DE LA SÉCHERESSE OCULAIRE

(30) Priority: 11.05.2017 CN 201710331802
(43) Date of publication of application: 15.04.2020
(73) Proprietor: Eye-medical (Xiamen) Biotechnology Co., Ltd., Xiamen City, Fujian Province 361000 (CN)
(72) Inventor: LIU, Zuguo, Xiamen Fujian 361000 (CN); ZHANG, Xiaobo, Xiamen Fujian 361000 (CN); LIN, Xiang, Xiamen Fujian 361000 (CN)
(74) Representative: Verscht, Thomas Kurt Albert
(86) International application number: PCT/CN2018/086240
(87) International publication number: WO 2018/205961

(56) References cited:
- EP-A1- 2 965 761
- CN-A- 101 528 217
- CN-A- 107 049 938
- JP-A- H07 215 881
- JP-A- H07 215 881
- SHINGO NAKAYAMADA ET AL: "Efficacy and safety of mizoribine for the treatment of Sjögren's syndrome: a multicenter open-label clinical trial", MODERN RHEUMATOLOGY, vol. 17, no. 6, 1 December 2007 (2007-12-01), pages 464-469, XP55752057, JP ISSN: 1439-7595, DOI: 10.1007/s10165-007-0627-2
- XIAOBO ZHANG et al.: "Topical Application of Mizoribine Suppresses CD4+ T- cell - Mediated Pathogenesis in Murine Dry Eye", Invest Ophthalmol Vis Sci., vol. 58, no. 14, 31 December 2017 (2017-12-31), pages 6056-6064, XP055612188, ISSN: 1552-5783

## Description

### FIELD OF THE DISCLOSURE

The present disclosure relates to the field of dry eye treating, and in particular relates to an eye drop for treating dry eye.

### BACKGROUND OF THE DISCLOSURE

Dry eye is a common and frequently-occurring disease in ophthalmology clinics. According to a large sample, dry eye epidemiological survey conducted by Schaumberg et al. in the United States, the prevalence of dry eye in women under 50 years old is 5.7%, and the prevalence rate increases to 9.8% over 75 years old. Dry eye symptoms are more pronounced in females than in men. The higher the level of education, the higher the prevalence rate. Similarly, the prevalence of dry eye in men aged 50-54 is 3.9%. When over 80 in age, the prevalence reaches 7.67%. It is estimated that by 2030, the number of male, dry eye patients in the United States will reach 2.79 million. In China, according to an epidemiological survey conducted by Shi Changhong et al., the dry eye prevalence rate was 67.9% among the 31,124 patients included, and the rate in females (72.7%) was higher than in male (62.8%). The incidence of dry eye in male subjects younger than 25 years old is higher than in female subjects. The prevalence of dry eye was 54.3% in the less than 25 years-old age group of, 65.9% in the 25-45 age group, and 79.1% in the over 45 years-old age group. With the development of science and technology, a large number of high-tech products have been widely used in the younger generation, such as mobile phones and tablet computers, which greatly increases the eye strain on young people. Coupled with the deterioration of the surrounding environment and the increase of life pressure, the prevalence rate of dry eye has increased year by year and has become significantly more prevalent in younger generations. Dry eye can cause dryness of the eyes, foreign body sensation, visual fatigue, decreased vision, etc. Serious dry eye can cause significant loss of vision and even blindness. The daily activities of dry eye patients such as reading, working, using computers, watching TV, and driving, are affected. It can be said that dry eye seriously affects human work efficiency and quality of life, and dry eye has become a social problem that modern people have to face and urgently need to solve.

The pathogenesis of dry eye is related to inflammation, apoptosis, neuromodulation abnormalities, and sex hormone disorders. The importance of inflammation in the pathogenesis of dry eye has been well documented in recent years. TNF-a, IL-1β, MMP-9, and other inflammatory factors are associated with ocular surface epithelial damage in dry eye patients. Inflammation of the ocular surface and lacrimal gland in patients with dry eye is primarily associated with massive infiltration and activation of *CD*4⁺*T* lymphocytes. Decreased goblet cells and squamous metaplasia were positively correlated with *CD*4⁺*T* cell infiltration and IFN-γ expression in dry eye patients. The inflammatory factors secreted by *CD*4⁺*T* cells play an important role in the development of dry eye. For example, the up-regulation of Th1 cytokine IFN-γ in dry eye ocular surface leads to a large number of apoptosis of conjunctival epithelial cells. The proportional up-regulation of IFN-γ/1L-13 leads to a decrease in the density of conjunctival goblet cells. The upregulation of the Th17 cytokine IL-17A results in damage to the corneal barrier function. Clinically, the expression of inflammatory markers, such as HLA class II, ICAM-1, and CCR5, in the conjunctival epithelium of patients with dry eye is significantly increased. Lam et al. recently confirmed that the expression of inflammatory chemokines IL-8, MIP-1α, and RANTES in tears of various dry eye patients is up-regulated, and *CD*11*b*⁺ and *CD*11c⁺ antigen-presenting cells are involved in dry eye ocular inflammation. The regulatory T cells play an important role in the pathogenesis of dry eye. *CD*4⁺, *CD*4⁺*T* cells, and *CD*8⁺ T cells can inhibit the increase of pathological *CD*4⁺*T* lymphocytes in dry eyes. NK/NKT cells can aggravate corneal barrier function by promoting dry eye Th-17 response. Clinical studies have shown that administration of anti-inflammatory drugs, such as corticosteroids, immunosuppressive cyclosporine, and FK506, applied topically to the eye can effectively alleviate symptoms and signs of dry eye.

In 2013, the Corneal Diseases Section of the Chinese Medical Association Ophthalmology Branch reached a consensus on dry eye clinical diagnosis and treatment, and established specifications for a dry eye definition, epidemiology and risk factors, classification, examination and diagnosis, and treatment. For moderate to severe dry eye, glucocorticoids, non-steroidal anti-inflammatory drugs, and immunosuppressive agents are recommended. Among them, immunosuppressive therapy is one of the keys to dry eye treatment. At present, the most commonly used immunosuppressive agents in clinical practice are 0.05% of cyclosporine A eye drops (Cyclosporine) and 0.05% tacrolimus eye drops (FK506). Both drugs showed good efficacy in clinical applications. After 6 months of topical treatment with cyclosporine A, the expression of inflammation-related markers (such as IL-1, TNF-α, etc.) decreased significantly. After 3 months of treatment with 0.05% of cyclosporine A, the ocular surface symptoms and signs were significantly reduced, and the inflammatory factors of IL-4, IL-5, IL-17A, TNFa, and IFN-γ in tears were significantly decreased. Topical treatment with cyclosporine A can effectively inhibit the infiltration of *CD*4⁺*T* cells, the apoptosis of conjunctival epithelial cells, and goblet cell loss in dry eye conjunctiva. Patients with Sjogren's syndrome use 0.03% of tacrolimus to improve tear film stability and maintain normal ocular surface condition. Animal experiments have shown that 0.025% of FK506 applied topically to the eye can inhibit the activation of NF-κB signaling pathway in mice and reduce the ocular surface signs of dry eye. 0.02% of tacrolimus drops applied topically in the eye can significantly increase the amount of tear secretion in dry eye experiments using dogs. However, cyclosporine A and FK506 are highly lipophilic and hardly soluble in water, and are mostly made into various vegetable oil eye drops prepared domestically and abroad. These oily eye drops are extremely irritating to the eyes, and the patient is usually intolerant of them. Occasionally, allergies occur, and the drug immediately separates from the tears, making the intraocular bioavailability low. In order to improve the intraocular bioavailability of the two drugs, preparation of the two drugs in microemulsions, micelles, liposomes, poly-e-caprolactone nanoparticles, chitosan nanoparticles, and the like have been studied. The above processes can effectively increase the drug loading of poorly soluble drugs or water insoluble drugs. In particular, the emulsification technique can reduce the irritation of the topical application, and as the dispersion of the emulsion droplets is large, the absorption of the medicine is fast and the bioavailability can be effectively improved. Even so, the congenital deficiency of fat-soluble eye drops is still not completely overcome at present. More than 1 billion people around the world suffer from dry eye, and there is a huge need for dry eye treatment. Therefore, it is extremely urgent to screen for new ocular surface immunosuppressants for dry eye, especially for water-soluble ophthalmic immunosuppressants.

### BRIEF SUMMARY OF THE DISCLOSURE

The present disclosure provides an eye drop for treating dry eye to solve deficiencies of the existing techniques.

A technical solution of the present disclosure is as follows: an eye drop for treating dry eye, a pH value of the eye drop is 5.0-7.0, the eye drop comprises 5-hydroxy-1-beta-D-furanosyl-1H-imidazole-5-carboxamide, (also named as mizoribine). The eye drop comprises the following components with the following weight percentages: 0.05∼0.1% of 5-hydroxy-1-beta-D-furanosyl-1H-imidazole-5-carboxamide, an appropriate amount of pH regulator, 0.01∼3% of an isotonic agent, 0.003∼0.5% of a bacteriostatic agent, 0.001∼0.5% of a stabilizer, 0.01∼0.5% of a tackifier, 2∼5% of a solubilizer, and a balance being water.

In another preferred embodiment, the pH regulator is at least one selected from a group consisting of sodium dihydrogen phosphate, disodium hydrogen phosphate, potassium dihydrogen phosphate, dipotassium hydrogen phosphate, boric acid, borax, acetic acid, sodium acetate, citric acid, sodium citrate, tartaric acid, sodium tartrate, sodium carbonate, potassium carbonate, sodium hydrogen carbonate, potassium hydrogen carbonate, sodium hydroxide, potassium hydroxide, hydrochloric acid, and phosphoric acid. The pH regulator makes the pH of the eye drop be equal to or close to a pH of tears and reduces an irritation of the eye drops, stabilizes the drug, and improves the effects of the drug.

In another preferred embodiment, the isotonic agent is at least one selected from sodium chloride, potassium chloride, boric acid, borax, sodium sulfate, potassium sulfate, sodium nitrate, potassium nitrate, sodium acetate, mannitol, glycerol, propylene glycol, or glucose. The isotonic agent makes an osmotic pressure of the eye drops equal to or close to the osmotic pressure of the tears, so as to reduce the irritation of the eye drops and improve the effects of the drug.

In another preferred embodiment, the bacteriostatic agent is at least one selected from a group consisting of benzalkonium chloride, benzalkonium bromide, chlorhexidine acetate, chlorhexidine glucose, chlorobutanol, phenoxyethanol, methylparaben, ethylparaben, and propylparaben. The bacteriostatic agent can make the eye drops to be not easily infected by bacteria, thereby ensuring the safety of the product.

In another preferred embodiment, the stabilizer is at least one selected from a group consisting of sodium sulfite, sodium bisulfite, sodium metabisulfite, sodium nitrite, sodium thiosulfate, ascorbic acid, thiourea, ascorbyl stearate, dibutyl cresol, cysteine, tocopheryl acetate, dichloroisocyanuate, disodium ethylenediaminetetraacetate, sodium calcium ethylenediaminetetraacetate, dimercaptopropanol, glycerol, mannitol, and butylated hydroxyanisole. The stabilizer is configured to increase a stability of the eye drop and improve the effects of the drugs.

In another preferred embodiment, the tackifier is at least one selected from a group consisting of sodium hyaluronate, carboxymethylcellulose sodium, Grade-A cellulose, polyethylene glycol, polyvinyl alcohol, carbomer, and povidone. The tackifier can increase a viscosity of the eye drops, prolong a retention time of the drug on the ocular surface, and increase an effective concentration of the drug.

In another preferred embodiment, the solubilizer is at least one selected from a group consisting of polysorbate, polyoxyethylene castor oil glyceryl ether, polyoxyethylene fatty acid ester, polyoxyethylene fatty alcohol ether, and poloxamer. The solubilizer can increase a solubility of the drug.

The beneficial effects of the present disclosure are as follows: the topical treatment of the eye drops of the present disclosure can protect the corneal epithelial barrier function of mice suffering dry eye, renew a number of conjunctival goblet cells, and have better anti-inflammatory and immunosuppressive effects.

### BRIEF DESCRIPTION OF THE DRAWING

FIG. 1 illustrates a graph showing a result of fluorescent staining of corneal OGD of embodiment 10 of the present disclosure.
FIG. 2 illustrates a graph showing a result of staining of conjunctival goblet cells PAS of embodiment 10 of the present disclosure.
FIG. 3 illustrates a graph showing a result of staining of conjunctival *CD*4⁺*T* cell of embodiment 10 of the present disclosure.
FIG. 4 illustrates a graph showing a result of Real-Time PCR of embodiment 10 of the present disclosure.

### DETAILED DESCRIPTION OF THE EMBODIMENTS

The present disclosure will be further described below with the combination of the accompanying drawings together with the embodiments.

### Embodiment 1

### Components:

| | |
|---|---|
| mizoribine | 1_{.}00g |
| benzalkonium chloride | 0.03g |
| sodium chloride | 8.30g |
| 0.1mol/L hydrochloric acid | 30mL |
| water for injection | constant volume to 1000mL |

### Preparation process:

1. Accurately weighting 1.00g of mizoribine, 0.03g of benzalkonium chloride, 8.30g of sodium chloride, and 30mL of 0.1mol/L hydrochloric acid, adding 200mL of water for injection, dissolving, stirring evenly, and adding water for injection until a volume of 1000mL is achieved to obtain a medicine solution.
2. Filtering the medicine solution once through a 0.45µm microporous membrane, then filtering twice through a 0.22µm microporous membrane.
3. After chemical tests of the medicine solution, in an A-level environment, filling 5.0mL of the medicine solution into a 5mL single-dose plastic container package, and sealing to obtain the product.

### Embodiment 2

### Components:

| | |
|---|---|
| mizoribine | 1.00g |
| chlorhexidine acetate | 0.05g |
| sodium dihydrogen phosphate | 7.20g |
| disodium hydrogen phosphate | 0.95g |
| sodium chloride | 4.79g |
| water for injection | constant volume to 1000mL |

### Preparation process:

1. Accurately weighting 1.00g of mizoribine, adding 300mL of water for injection, and dissolving to obtain a first solution for later use.
2. Accurately weighting 0.05g of chlorhexidine acetate, 7.20g of sodium dihydrogen phosphate, 0.95g of disodium hydrogen phosphate, and 4.79g of sodium chloride, dissolving in 200mL of water for injection, and then stirring and mixing evenly to obtain a second solution for later use.
3. Mixing the first solution and the second solution, stirring evenly, and adding water for injection until a volume of 1000mL is achieved to obtain a medicine solution.
4. Filtering the medicine solution once through a 0.45µm microporous membrane, then filtering twice through a 0.22µm microporous membrane.
5. After chemical tests of the medicine solution, in the A-level environment, filling 5.0mL of the medicine solution into a 5mL single-dose plastic container package, sealing to obtain the product.

### Embodiment 3

### Components:

| | |
|---|---|
| mizoribine | 1.00g |
| benzalkonium bromide | 0.03g |
| sodium dihydrogen phosphate | 6.40g |
| disodium hydrogen phosphate | 1.89g |
| sodium chloride | 4.72g |
| disodium ethylenediaminetetraacetate | 0.10g |
| water for injection | constant volume to 1000mL |

### Preparation process:

1. Accurately weighting 1.00g of mizoribine, 0.03g benzalkonium bromide, 6.40g of sodium dihydrogen phosphate, 1.89g of disodium hydrogen phosphate, 4.72g of sodium chloride, and 0.10g of disodium ethylenediaminetetraacetate, adding 200mL of water for injection, dissolving, stirring evenly, and adding water for injection until a volume of 1000mL is achieved to obtain a medicine solution.
2. Filtering the medicine solution once through a 0.45µm microporous membrane, and then filtering twice through a 0.22µm microporous membrane.
3. Sterilizing the filtered medicine solution by autoclaving at 121°C for 8 minutes and cooling.
4. After chemical tests of the medicine solution, in the A-level environment, filling 5.0mL of the medicine solution into a 5mL single-dose plastic container package, and sealing to obtain the product.

### Embodiment 4

### Components:

| | |
|---|---|
| mizoribine | 1.00g |
| chlorhexidine glucose | 0.10g |
| boric acid | 19.10g |
| glycerol | 13.00g |
| water for injection | constant volume to 1000mL |

### Preparation process:

1. Accurately weighting 1.00g of mizoribine, 0.10g of chlorhexidine glucose, 19.10g of boric acid, and 13.00g of glycerol, adding 200mL of water for injection, dissolving, stirring evenly, and adding water for injection until a volume of 1000mL is achieved to obtain a medicine solution.
2. Filtering the medicine solution once through a 0.45µm microporous membrane, then filtering twice through a 0.22µm microporous membrane.
3. After chemical tests of the medicine solution, in the A-level environment, filling 5.0mL of the medicine solution into a 5mL single-dose plastic container package, sealing to obtain the product.

### Embodiment 5

### Components:

| | |
|---|---|
| mizoribine | 1.00g |
| benzalkonium chloride | 0.03g |
| sodium dihydrogen phosphate | 7.20g |
| disodium hydrogen phosphate | 0.95g |
| glycerol | 14.00g |
| disodium ethylenediaminetetraacetate | 0.10g |
| water for injection | constant volume to 1000mL |

### Preparation process:

1. Accurately weighting 1.00g of mizoribine, 0.03g of benzalkonium chloride, 7.20g of sodium dihydrogen phosphate, 0.95g of disodium hydrogen phosphate, 14.0g of glycerol, and 0.10g of disodium ethylenediaminetetraacetate, adding 200mL of water for injection, dissolving, stirring evenly, and adding water for injection until a volume of 1000mL is achieved to obtain a medicine solution.
2. Filtering the medicine solution once through a 0.45µm microporous membrane, then filtering once through a 0.22µm microporous membrane.
3. Sterilizing the filtered medicine solution by autoclaving at 121°C for 8 minutes and cooling.
4. After chemical tests of the medicine solution, in the A-level environment, filling 5.0mL of the medicine solution into a 5mL single-dose plastic container package, and sealing to obtain the product.

### Embodiment 6

### Components:

| | |
|---|---|
| mizoribine | 1.00g |
| benzalkonium chloride | 0.03g |
| sodium dihydrogen phosphate | 7.20g |
| disodium hydrogen phosphate | 0.95g |
| sodium chloride | 4.79g |
| disodium ethylenediaminetetraacetate | 0.10g |
| sodium hyaluronate | 0.10g |
| water for injection | constant volume to 1000mL |

### Preparation process:

1. Accurately weighting 0.10 g of sodium hyaluronate, adding 200mL of water for injection, swelling, and resting overnight while stirring for 5 min every 1 h.
2. Accurately weighting 1.00g of mizoribine, 0.03g of benzalkonium chloride, 7.20g of sodium dihydrogen phosphate, 0.95g of disodium hydrogen phosphate, 4.79g of sodium chloride, and 0.10g of disodium ethylenediaminetetraacetate, adding 200mL of water for injection, dissolving, stirring evenly, adding the sodium hyaluronate solution obtained in step 1, and then adding water for injection to until a volume of 1000mL is achieved to obtain a medicine solution.
3. Filtering the medicine solution once through a 0.45µm microporous membrane, and then filtering twice through a 0.22µm microporous membrane.
4. After chemical tests of the medicine solution, in the A-level environment, filling 5.0mL of the medicine solution into a 5mL single-dose plastic container package, and sealing to obtain the product.

### Embodiment 7

### Components:

| | |
|---|---|
| mizoribine | 1.00g |
| benzalkonium chloride | 0.03g |
| sodium dihydrogen phosphate | 6.40g |
| disodium hydrogen phosphate | 1.90g |
| sodium chloride | 4.70g |
| disodium ethylenediaminetetraacetate | 0.10g |
| sodium hyaluronate | 0.10g |
| water for injection | constant volume to 1000mL |

### Preparation process:

Accurately weighting 0.10 g of sodium hyaluronate, adding 200mL of water for injection, swelling, resting for 12h while stirring for 5 min every 1 h.
2. Accurately weighting 0.03g of benzalkonium chloride and 0.10g of disodium ethylenediaminetetraacetate, adding 300mL of water for injection, dissolving, stirring evenly, then accurately weighting 6.40g of sodium dihydrogen phosphate, 1.90g of disodium hydrogen phosphate, and 4.70g of sodium chloride to the above solution, and stirring evenly.
3. Accurately weighting 1.00g of mizoribine to the mixed solution obtained in step 2, heating appropriately during a dissolving process, dissolving while stirring, and cooling to room temperature if heated.
4. Adding the solution obtained in the step 3 to the dissolved sodium hyaluronate solution obtained in the step 1, stirring and mixing evenly, and then adding water for injection to until a volume of 1000mL is achieved to obtain a medicine solution.
5. Filtering the medicine solution once through a 0.45µm microporous membrane, and then filtering twice through a 0.22µm microporous membrane.
6. After chemical tests of the medicine solution, in the A-level environment, filling 5.0mL of the medicine solution into a 5mL single-dose plastic container package, and sealing to obtain the product.

### Embodiment 8

### Components:

| | |
|---|---|
| mizoribine | 1.00g |
| benzalkonium bromide | 5.00g |
| sodium dihydrogen phosphate | 6.40g |
| disodium hydrogen phosphate | 1.90g |
| sodium chloride | 4.70g |
| sodium sulfite | 5.00g |
| sodium hyaluronate | 0.10g |
| water for injection | constant volume to 1000mL |

### Preparation process:

1. Accurately weighting 0.10 g of sodium hyaluronate, adding 200mL of water for injection, swelling, and resting for 12h while stirring for 5 min every 1 h.
2. Accurately weighting 5.00g of benzalkonium bromide and 5.00g of sodium sulfite, adding 500mL of water for injection, dissolving, stirring evenly, then accurately weighting 6.40g of sodium dihydrogen phosphate, 1.90g of disodium hydrogen phosphate, and 4.70g of sodium chloride to the above solution, and stirring evenly.
3. Accurately weighting 1.00g of mizoribine to the mixed solution obtained in step 2, heating appropriately during a dissolving process, stirring, and cooling to room temperature if heated.
4. Adding the solution obtained in the step 3 to the dissolved sodium hyaluronate solution obtained in the step 1, stirring and mixing evenly, and then adding water for injection to until volume of 1000mL is achieved to obtain a medicine solution.
5. Filtering the medicine solution once through a 0.45µm microporous membrane, and then filtering twice through a 0.22µm microporous membrane.
6. After chemical tests of the medicine solution, in the A-level environment, filling 5.0mL of the medicine solution into a 5mL single-dose plastic container package, and sealing to obtain the product.

### Embodiment 9

### Components:

| | |
|---|---|
| mizoribine | 1.00g |
| benzalkonium chloride | 0.03g |
| sodium dihydrogen phosphate | 6.40g |
| disodium hydrogen phosphate | 1.90g |
| sodium chloride | 4.70g |
| disodium ethylenediaminetetraacetate | 0.10g |
| sodium hyaluronate | 0.10g |
| polysorbate 80 | 30mL |
| water for injection | constant volume to 1000mL |

### Preparation process:

1. Accurately weighting 0.10g of sodium hyaluronate, adding 200mL of water for injection, swelling, and resting for 12h while stirring for 5 min every 1 h.
2. Accurately weighting 6.40g of sodium dihydrogen phosphate and 1.90g of disodium hydrogen phosphate to 300mL of water for injection and stirring evenly.
3. Accurately weighting 1.00g of mizoribine and 4.70g of sodium chloride, grinding into a fine powder, adding 30mL of polysorbate 80, and mixing evenly. Accurately weighting 0.03g of benzalkonium chloride and 0.10g of disodium ethylenediaminetetraacetate, adding 300mL of water for injection, dissolving, heating and stirring, maintaining a water temperature at 70-80 °C, and completely transferring the mizoribine et al. into the above aqueous solution while stirring evenly, adding 300mL of water for injection, and cooling to room temperature to clarify.
4. Adding drop-by-drop the solution obtained in the step 3 to the solution obtained in the step 2, then slowly adding the dissolved sodium hyaluronate solution, stirring and mixing evenly, and then adding water for injection until a volume of 1000mL is achieved to obtain a medicine solution.
5. Filtering the medicine solution once through a 0.45µm microporous membrane, and then filtering twice through a 0.22µm microporous membrane.
6. After chemical tests of the medicine solution, in the A-level environment, filling 5.0mL of the medicine solution into a 5mL single-dose plastic container package, and sealing to obtain the product.

In the aforementioned embodiments, an amount of prescription preparation are all described by way of 1000mL. In actual industrial production, the amount of prescription preparation can be increased according to a proportion of the prescription.

### Embodiment 10: Ocular efficacy evaluation

Therapeutic effect of 0.1% of mizoribine eye drop on mice suffering dry eye

### Experimental method:

1. Establish a dry eye mouse model: Female C57BL/6 mice of 10-12 weeks old were injected subcutaneously with scopolamine 0.5 mg/0.2mL four times a day and kept in a dry environment for 5 days (RH: 30±3; T: 23-25°C); normal control group was maintained in normal environment (RH: 60% - 80%; T: 23-25°C).

Preparation of mizoribine eye drop: dry eye treatment group used 0.1% of mizoribine eye drop prepared by the method of Embodiment 1.

Eye drop method: Eye drop treatment was performed using the above-mentioned eye drops 4 times a day on both eyes.

### Evaluation indicators and methods of operation:

1. Corneal OGD fluorescent staining: mice were sacrificed, and 0.5µl of Oregongreen-dextran (OGD) was dropped into the conjunctival sac of mice with a pipette, manual blinking was performed, and the conjunctival sac was washed with 1mL of normal saline 1 minute later. The corneal epithelial fluorescence staining was observed and shot under a living fluorescence microscope.
2. PAS staining of conjunctival goblet cells: Paraffin specimens of the whole eyeballs (conjunctival sac) of the mice were cut into 5um sections and stained with periodic acid-Schiff (PAS) reagent to observe the morphology and number of conjunctival goblet cells.
3. Conjunctival *CD*4⁺*T* cell staining: fixing frozen sections by acetone at -20°C for 10 minutes, disrupting with 0.2% of Triton-X PBS for 10 minutes, and using 0.3% of H₂O₂ to eliminate endogenous peroxidase, blocking with 20% of normal goat serum for 45 minutes. The primary anti-CD4 was incubated for 1 hour at room temperature. After extensive washing (digestion), the biotinylated (second) antibody was incubated for 30 minutes. Finally, a Vectastain Elite ABC kit was used to develop color and stain the nucleus.
4. Real-Time PCR :

### Prepare the PCR reaction solution according to the following components:

| | |
|---|---|
| DEPC+ conjunctival RNA | 12 |
| 40u RNase inhibitor (may be not added) | 0.5 |
| 5×Reaction Buffer | 4 |
| 10mM dNTPs | 2 |
| Random Primer | 1 |
| RT enzyme | 1 |
| Total | 20 |

Three-step PCR amplification standard procedure:
Stage 1: Pre-denaturation cycle number: 195 °C, 30 sec.
Stage 2: number of PCR reaction cycles: 4095 °C for 5 sec. and 60 ° C for 34 sec.
Stage 3: Dissolving curve

The relative expression level of the gene was analyzed by the relative Ct method, and the specific expression of the target gene was calculated by the following formula, that is to say, the relative expression level= 2^{-ΔΔ*Ct*} ; wherein *-*ΔΔ*Ct* = -(Δ*Ct, q -* Δ*Ct*, *cb*), Δ*Ct,* q *= Ct,x* - *Ct,r ,* Δ*Ct, cb = Ct, cb - Ct, r*, where r=reference gene, Cb=calibration value, X=target gene, and cycle threshold (C) is determined through primary signal (fluorescence).

### Experimental results and conclusions:

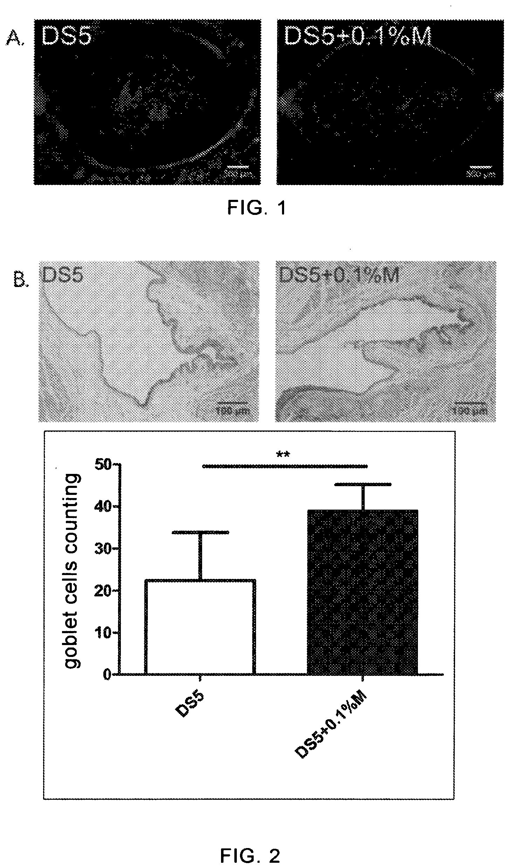

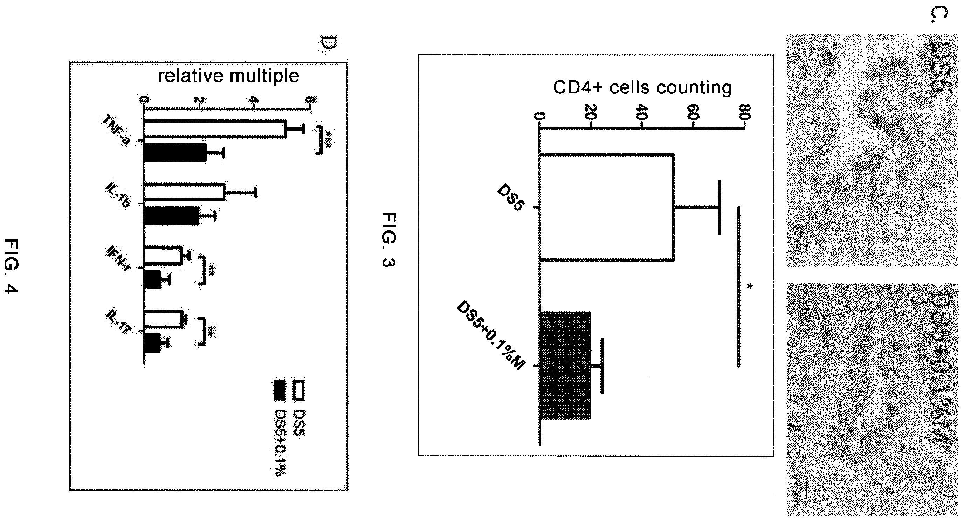

1. Corneal OGD fluorescence staining results showed that the number of corneal punctiform staining was significantly reduced in the 0.1% of mizoribine group compared with the dry eye group, and no flaky staining was observed. The results showed that 0.1% of mizoribine topical eye treatment can significantly reduce corneal epithelial damage and have the effect of protecting the corneal epithelial barrier (as shown in FIG. 1)
2. PAS staining of conjunctival goblet cells showed a significant increase in the number of conjunctival goblet cells in the 0.1% of mizoribine group compared with the dry eye group. The results showed that: 0.1% of mizoribine topical eye treatment can significantly restore the number of goblet cells and ensure the amount of mucin secretion (as shown in FIG. 2).
3. Conjunctival *CD*4⁺*T* cell staining results showed that the proportion of brown-yellow cells in the 0.1% mizoribine treatment group was significantly reduced compared with the dry eye group. Statistical analysis of the *CD*4⁺*T* cell counts of the two groups showed that the difference is statistically significant. The results showed that 0.1% of mizoribine topical eye treatment can significantly reduce the conjunctival *CD*4⁺*T* cell infiltration, with obvious anti-inflammatory effect (As shown in FIG. 3).
4. Real-Time PCR results showed that compared with the dry eye group, the expression of conjunctival inflammatory factors TNF-α, IL-1β, IFN-γ and IL-13 was significantly decreased in the 0.1% mizoribine treatment group, and the difference is statistically significant. The results showed that 0.1% of mizoribine topical eye treatment has better anti-inflammatory and immunosuppressive effects (As shown in FIG. 4).

It will be apparent to those skilled in the art that various modifications and variation can be made in the present disclosure without departing from the spirit or scope of the invention. Thus, it is intended that the present disclosure cover the modifications and variations of this invention provided they come within the scope of the appended claims and their equivalents.

## Claims

1. An eye drop for treating dry eye, **characterized in that**:
a pH value of the eye drop is 5.0-7.0,
the eye drop comprises 5-hydroxy-1-beta-D-furanosyl-1H-imidazole-5-carboxamide, and
the eye drop comprises the following components with the following weight percentage:
0.05 ∼ 0.1% of the 5-hydroxy-1-beta-D-furanosyl-1H-imidazole-5-carboxamide, an appropriate amount of a pH regulator,
0.01 ∼ 3% of an isotonic agent,
0.003 ∼ 0.5% of a bacteriostatic agent,
0.001 ∼ 0.5% of a stabilizer,
0.01 ∼ 0.5% of a tackifier,
2 ∼ 5% of a solubilizer, and
a balance being water.

2. The eye drop for treating dry eye according to claim 1, **characterized in that**:
the pH regulator is at least one selected from a group consisting of sodium dihydrogen phosphate, disodium hydrogen phosphate, potassium dihydrogen phosphate, dipotassium hydrogen phosphate, boric acid, borax, acetic acid, sodium acetate, citric acid, sodium citrate, tartaric acid, sodium tartrate, sodium carbonate, potassium carbonate, sodium hydrogen carbonate, potassium hydrogen carbonate, sodium hydroxide, potassium hydroxide, hydrochloric acid, and phosphoric acid.

3. The eye drop for treating dry eye according to claim 1, **characterized in that**:
the isotonic agent is at least one selected from sodium chloride, potassium chloride, boric acid, borax, sodium sulfate, potassium sulfate, sodium nitrate, potassium nitrate, sodium acetate, mannitol, glycerol, propylene glycol, or glucose.

4. The eye drop for treating dry eye according to claim 1, **characterized in that**:
the bacteriostatic agent is at least one selected from a group consisting of benzalkonium chloride, benzalkonium bromide, chlorhexidine acetate, chlorhexidine glucose, chlorobutanol, phenoxyethanol, methylparaben, ethylparaben, and propylparaben.

5. The eye drop for treating dry eye according to claim 1, **characterized in that**:
the stabilizer is at least one selected from a group consisting of sodium sulfite, sodium bisulfite, sodium metabisulfite, sodium nitrite, sodium thiosulfate, ascorbic acid, thiourea, ascorbyl stearate, dibutyl cresol, cysteine, tocopheryl acetate, dichloroisocyanuate, disodium ethylenediaminetetraacetate, sodium calcium ethylenediaminetetraacetate, dimercaptopropanol, glycerol, mannitol, and butylated hydroxyanisole.

6. The eye drop for treating dry eye according to claim 1, **characterized in that**:
the tackifier is at least one selected from a group consisting of sodium hyaluronate, carboxymethylcellulose sodium, Grade-A cellulose, polyethylene glycol, polyvinyl alcohol, carbomer, and povidone.

7. The eye drop for treating dry eye according to claim 1, **characterized in that**:
the solubilizer is at least one selected from a group consisting of polysorbate, polyoxyethylene castor oil glyceryl ether, polyoxyethylene fatty acid ester, polyoxyethylene fatty alcohol ether, and poloxamer..

## Patentansprüche

1. Ein Augentropfen zur Behandlung von trockenen Augen, **dadurch gekennzeichnet, dass**:
der pH-Wert des Augentropfens 5,0-7,0 beträgt,
der Augentropfen 5-Hydroxy-1-beta-D-furanosyl-1H-imidazol-5-carboxamid umfasst und der Augentropfen die folgenden Komponenten mit den folgendem Gewichtspro-zentanteilen umfasst:
0,05 ∼ 0,1% des 5-Hydroxy-1-beta-D-furanosyl-1H-imidazol-5-carboxamids, eine geeignete Menge eines pH-Regulators,
0,01 ∼ 3% eines isotonischen Mittels,
0,003 ∼ 0,5% eines bakteriostatischen Mittels,
0,001 ∼ 0,5% eines Stabilisators,
0,01 ∼ 0,5% eines Klebrigmachers,
2 ∼ 5% eines Losungsvermittlers und wobei
der Rest Wasser ist.

2. Der Augentropfen zur Behandlung von trockenen Augen nach Anspruch 1, **dadurch gekennzeichnet, dass**:
der pH-Regulator wenigsten einer ist, welcher aus einer Gruppe bestehend aus Natriumdihydrogenphosphat, Dinatriumhydrogenphosphat, Kaliumdihydrogenphosphat, Dikaliumhydrogenphosphat, Borsäure, Borax, Essigsäure, Natriumacetat, Citronensäure, Natriumcitrat, Weinsäure, Natriumtartrat. Natriumcarbonat, Kaliumcarbonat, Natriumhydrogenearbonat, Kaliumhydrogencarbonat, Natriumhydroxid, Kaliumhydroxid, Salzsäure und Phosphorsäure ausgewählt ist.

3. Der Augentropfen zur Behandlung von trockenen Augen nach Anspruch 1, **dadurch gekennzeichnet, dass**:
das isotonische Mittel wenigstens eines ist, welches aus Natriumchlorid, Kaliumchlorid, Borsäure, Borax, Natriumsulfat, Kaliumsulfat, Natriumnitrat, Kaliumnitrat, Natriumacetat, Manitol, Glycerin, Propylenglycol oder Glucose ausgewählt ist.

4. Der Augentropfen zur Behandlung von trockenen Augen nach Anspruch 1, **dadurch gekennzeichnet, dass**:
das bakteriostatische Mittel wenigstens eines ist, welches aus einer Gruppe bestehend aus Benzalkoniumchlorid, Benzalkoniumbromid, Chlorhexidinacetat, Chlorhexidinglucose, Chlorbutanol, Phenoxyethanol, Methylparaben, Ethylparaben und Propylparaben ausgewählt ist.

5. Der Augentropfen zur Behandlung von trockenen Augen nach Anspruch 1, **dadurch gekennzeichnet, dass**:
der Stabilisator wenigsten einer ist, welcher aus einer Gruppe bestehend aus Natriumsulfit, Natriumbisulfit, Natriummetabisulfit, Natriumnitrit, Natriumthiosulfat, Ascorbinsäure, Thioharnstoff, Ascorbylstearat , Dibutyleresol, Cystein, Tocopherylacetat, Dichlorisocyanuat, Dmatriumethylendiamintetraacetat, Natriumcalciumethylendiamintetraacetat, Dimercaptopropanol, Glycerin, Manitol und butyliertem Hydroxyanisol ausgewählt ist.

6. Der Augentropfen zur Behandlung von trockenen Augen nach Anspruch 1, **dadurch gekennzeichnet, dass**:
der Klebrigmacher wenigsten einer ist, welcher aus einer Gruppe bestehend aus Natriumhyaluronat, Carboxymethylcellulose-Natrium, Grad-A-Cellulose, Polyethylenglycol, Polyvinylalkohol, Carbomer und Povidon ausgewählt ist.

7. Der Augentropfen zur Behandlung von trockenen Augen nach Anspruch 1, **dadurch gekennzeichnet, dass**;
der Lösungsvermittler wenigsten einer ist, welcher aus einer Gruppe bestehend aus Polysorbat, Polyoxyethylen-Rizinusöl-Glycerylether, Polyoxyethylen-Fettsäureester, Polyoxyethylen-Fettalkoholether und Poloxamer ausgewählt ist.

## Revendications

1. Goutte ophtalmique pour le traitement d'une sécheresse oculaire, **caractérisée en ce que** :
la valeur de pH de la goutte ophtalmique est de 5,0 à 7,0,
la goutte ophtalmique comprend du 5-hydroxy-1-bêta-D-furanosyl-1H-imidazole-5-carboxamide, et
la goutte ophtalmique comprend les composants suivants en les pourcentages en poids suivants ;
0,05 à 0,1 % du 5-hydroxy-1-bêta-D-furanosyl-1H-imidazole-5-carboxamide,
une quantité appropriée d'un régulateur de pH,
0,01 à 3 % d'un agent isotonique,
0,003 à 0,5 % d'un agent bactériostatique,
0,001 à 0,5 % d'un stabilisant,
0,01 à 0,5 % d'un agent conférant du collant,
2 à 5 % d'un solubilisant, et
le reste étant de l'eau.

2. Goutte ophtalmique pour le traitement d'une sécheresse oculaire selon la revendication 1, **caractérisée en ce que** :
le régulateur de pH est au moins l'un choisi dans le groups constitué par le dihydrogénophosphate de sodium, l'hydrogénophosphate disodique, le dihydrogénophosphate de potassium, l'hydrogénophosphate dipotassique, l'acide borique, le borax, l'acide acetique, l'acétate de sodium, l'acide citrique, le citrate de sodium, l'acide tartrique, le tartrate de sodium, le carbonate de sodium, le carbonate de potassium, l'hydrogénocarbonate de sodium, l'hydrogénocarbonate de potassium, l'hydroxyde de sodium, l'hydroxyde de potassium, l'acide chlorhydrique, et l'acide phosphorique.

3. Goutte ophtalmique pour le traitement d'une sécheresse oculaire selon la revendication 1, **caractérisée en ce que** :
l'agent isotonique est au moins l'un choisi parmi le chlorure de sodium, le chlorure de potassium, l'acide borique, le borax, le sulfate de sodium, le sulfate de potassium, le nitrate de sodium, le nitrate de potassium, l'acétate de sodium, le mannitol, le glycérol, le propyleneglycol, et le glucose.

4. Goutte ophtalmique pour le traitement d'une sécheresse oculaire selon la revendication 1, **caractérisée en ce que** :
l'agent bactériostatique est au moins l'un choisi dans le groupe constitué par le chlorure de benzalkonium, le bromure de benzalkonium, l'acétate de chlorhexidine, le chlorhexidine-glucose, le chlorobutanol, le phenoxyethanol, le methylparaben, l'ethylparaben, et le propylparaben.

5. Goutte ophtalmique pour le traitement d'une sécheresse oculaire selon la revendication 1, **caractérisée en ce que** :
le stabilisant est au moins l'un choisi dans le groupe constitué par le sulfite de sodium, le bisulfite de sodium, le métabisulfite de sodium, le nitrite de sodium, le thiosulfate de sodium, l'acide ascorbique, la thiourée, le stéarate d'ascorbyle, le dibutyl-cresol, la cystéine, l'acétate de tocophéryle, le dichloroisocyanurate, l'éthylènediaminetetraacétate disodique, l'éthylènediaminetétraacétate de sodium et de calcium, le dimercaptopropanol, le glycerol, le mannitol, et l'hydroxyanisole butylé.

6. Goutte ophtalmique pour le traitement d'une sécheresse oculaire selon la revendication 1, **caractérisée en ce que** :
l'agent conférant du collant est au moins l'un choisi dans le groupe constitué par le hyaluronate de sodium, la carboxyméthylcellulose sodique, la cellulose de grade A, le polyéthylèneglycol, le poly(alcool vinylique), un carbomère, et la povidone.

7. Goutte ophtalmique pour le traitement d'une sécheresse oculaire selon la revendication 1, **caractérisée en ce que** :
le solubilisant est au moins l'un choisi dans le groupe constitué par un polysorbate, l'éther glycérylique d'huile de ricin polyoxyéthyléné, un ester d'acide gras polyoxyéthyléné, un éther d'alcool gras polyoxyéthyléné, et un poloxamère.
